# FASCICULE DE BREVET EUROPEEN

(11) **EP 0 524 892 B1**
(45) Date de publication et mention de la délivrance du brevet: **01.03.1995**
(21) Numéro de dépôt: 92402151.2
(22) Date de dépôt: 24.07.1992
(51) Int. Cl.: A61K 7/48, B01F 3/12

(54) **Procédé de préparation d'une dispersion solide anhydre**
Verfahren zur Herstellung einer wasserfreien Feststoffdispersion
Method for preparation of an anhydrous solid dispersion

(30) Priorité: 26.07.1991 FR 9109514
(43) Date de publication de la demande: 27.01.1993
(73) Titulaire: L'OREAL, 75008 Paris (FR)
(72) Inventeur: Ser, Jean-Claude, F-94150 Chevilly-Larue (FR); Miguel, Dolorès, F-93300 Aubervilliers (FR)
(74) Mandataire: Stalla-Bourdillon, Bernard

(56) Documents cités:
- FR-A- 2 237 615
- PATENT ABSTRACTS OF JAPAN vol. 13, no. 399 (C-632)(3747) 5 Septembre 1989
- PATENT ABSTRACTS OF JAPAN vol. 8, no. 266 (C-255)(1703) 6 Décembre 1984
- PATENT ABSTRACTS OF JAPAN vol. 15, no. 238 (C-841)(4766) 19 Juin 1991

## Description

La présente invention a pour objet un procédé de préparation d'une dispersion solide contenant à l'état dispersé une quantité importante d'alcool polyhydrique ("polyol") dans un corps gras, ladite dispersion obtenue étant destinée à un usage cosmétique ou pharmaceutique.

Les produits cosmétiques ou pharmaceutiques gras solides habituellement utilisés notamment sous forme de bâtons, par exemple de rouge à lèvres, présentent l'inconvénient de ne pas être hydratants.

Pour pallier cet inconvénient, on a tenté d'y introduire de l'eau en préparant des produits émulsionnés.

On peut à cet égard citer la demande de brevet français n° 73 26446 (2.237.615) qui décrit une émulsion eau-dans-l'huile contenant de 1 à 50 % en poids d'eau.

Toutefois, ce type de composition pose des problèmes d'évaporation de l'eau.

La composition décrite dans cette demande de brevet contient également de 1 à 10 % d'un composé polyhydroxylé afin d'obtenir une répartition uniforme des substances colorantes dans l'émulsion eau-dans-l'huile.

Le problème de l'évaporation de l'eau des dispersions solides a été résolu selon la demande de brevet européen n° EP 374.332 par l'incorporation d'une huile siliconée ainsi que d'un organopolysiloxane modifié par un polyoxyalkylène.

Les compositions ainsi modifié es peuvent également contenir des polyols en faible quantite en tant qu'humectants additionnels.

Cependant, aucun produit contenant de l'eau en quantité conséquente n'a encore été commercialisé jusqu'à présent du fait justement des problèmes de perte d'eau qui semblent ne pas encore avoir été résolus de manière satisfaisante.

Selon la demande de brevet japonais n° (de publication) 1-143.812, il est décrit l'incorporation d'une quantité importante d'alcools polyvalents dans une émulsion solide à usage cosmétique par le biais d'une huile de silicone et d'un organopolysiloxane de polyoxyalkylène modifié en tant qu'agent émulsifiant.

Or, avec ce système, il est difficile d'obtenir une bonne homogénéité de la formule.

Un but de la présente invention est de pallier les inconvénients décrits ci-dessus.

Suite à diverses études, il a été constaté d'une manière inattendue et surprenante qu'une quantité importante d'alcool polyhydrique pouvait être incorporée dans une dispersion solide grâce à l'utilisation, pendant l'incorporation, d'une turbine à une vitesse de rotation minimum ce qui produit des particules de très faible taille.

L'invention a donc pour objet un procédé pour la préparation d'une dispersion solide anhydre et stable comprenant de 20 à 95 % et de préférence de 40 à 85 % d'un corps gras constitué de 10 à 50 % d'au moins une cire de point de fusion supérieur à 55°C et de 4 à 50 % d'un alcool polyhydrique dispersé, de préférence de 6 à 40 %, et plus particulièrement de 8 à 25 %, caractérisé en ce que l'on chauffe ledit corps gras et l'alcool polyhydrique à une température comprise entre 65 et 95°C et que l'on procède au mélange à l'aide d'une turbine ayant une vitesse de rotation d'au moins 1500 tours par minute (t/mn) et de préférence, comprise entre 2500 et 3500 t/mn.

On entend par l'expression "dispersion solide" une composition solide entre 0 et 50°C, ce qui correspond au domaine de température de stockage et d'utilisation des produits cosmétiques.

L'invention a également pour objet une dispersion solide anhydre et stable comprenant de 20 à 95 % d'un corps gras constituant la phase continue et de 4 à 50 %, de préférence de 6 à 40 %, et plus particulièrement de 8 à 25 %, d'un alcool polyhydrique dispersé caractérisée en ce que la taille moyenne des particules d'alcool polyhydrique est inférieure ou égale à 1 »m et de préférence comprise entre 0,01 et 0,8 »m et en ce que le corps gras est constitué de 10 à 50 % en poids d'au moins une cire de point de fusion supérieur à 55°C.

La dispersion selon l'invention permet d'obtenir des produits cosmétiques possédant d'excellentes propriétés émollientes par application sur la peau.

Les dispersions solides selon l'invention s'appliquent aux produits gras coulés comme les rouges à lèvres, fonds de teint, fards à paupières, fards à joues et notamment aux produits sous forme de bâtons.

Selon une forme de réalisation préférée, la dispersion solide selon l'invention peut également contenir jusqu'à 10 % d'une charge minérale ou organique telle que le talc, l'amidon, etc.

Il est également possible que la dispersion selon l'invention contienne jusqu'à environ 30 % (de préférence de 0,1 à 20 %) d'un pigment et de 0,01 à 20 % (de préférence de 0,5 à 10 %) d'un agent tensioactif hydrocarboné. L'emploi d'un tensioactif hydrocarboné permet d'obtenir une dispersion plus fine et donc un bâton plus satisfaisant.

Selon un mode de réalisation plus particulièrement préféré, il est possible d'ajouter à la dispersion selon l'invention une quantité suffisante d'un polymère pour la stabiliser davantage en atmosphère humide.

De tels polymères doivent être liposolubles et posséder un taux de motifs hydrophiles très faible.

Parmi ceux-ci, on peut citer les polyalkylènes (notamment les polyéthylènes et polybutènes), les polyacrylates et les polymères siliconés compatibles avec les corps gras.

Parmi les polyalkylènes, on peut citer le polybutène, notamment celui vendu par la Société Amoco sous la dénomination INDOPOL.

Le rapport en poids du polymère à l'alcool polyhydrique est généralement inférieur à 4 et est de préférence compris entre 0,25 et 2.

Selon l'invention, l'alcool polyhydrique peut être un composé ayant de 2 à 8 atomes de carbone et de 2 à 6 fonctions hydroxy. Parmi ces composés, on peut citer : l'éthylène glycol, la glycérine, le propanediol-1,2, la diglycérine, l'érythritol, l'arabitol, l'adonitol, le sorbitol et le dulcitol.

L'alcool polyhydrique peut être également un polyéther alcool de poids moléculaire moyen compris entre 150 et 600 et parmi ceux-ci on peut citer le polyéthylène glycol 300 et la polyglycérine 500.

L'alcool polyhydrique peut être enrichi éventuellement d'actifs hydrosolubles tels que les acides aminés (par exemple l'arginine, la lysine, la proline et la sérine), les vitamines telles que le D,L Panthénol et les filtres solaires. Ceux-ci peuvent être présents à raison de 0,05 % à 5 %.

Comme indiqué précédemment, le corps gras selon l'invention est constitué de 10 à 50 % en poids d'au moins une cire dont le point de fusion est supérieur à 55°C, le reste étant soit une cire de point de fusion inférieur à 55°C soit une huile ou un mélange de ceux-ci. Le point de fusion finissant de tout mélange doit être inférieur à 110°C ce qui n'empêche pas que certains constituants du mélange puissent présenter un point de fusion supérieur.

Parmi les cires, ayant un point de fusion supérieur à 55°C, susceptibles d'être utilisées selon l'invention, on peut mentionner les cires animales, végétales, minérales, synthétiques et les fractions diverses de cires naturelles, toutes ces cires ayant, en règle générale, un point de fusion compris entre 55 et 110°C, et une pénétration à l'aiguille, à 25°C, comprise entre environ 3 et 40, telle que mesurée selon la norme américaine ASTM D5 ou selon la norme française NFT 004. Le principe de la mesure de la pénétration d'une aiguille selon ces deux normes consiste à mesurer la profondeur, exprimée en dixièmes de millimètre, à laquelle pénètre une aiguille normalisée (pesant 2,5 g, placée dans un porte-aiguille pesant 47,5 g, soit au total, 50 g), placée sur la cire pendant 5 secondes.

Parmi les cires animales que l'on peut utiliser, on peut citer entre autres les cires d'abeille, les cires de lanoline, les cires d'insecte de chine et les dérivés issus de la lanoline. Parmi les cires végétales, on peut citer, entre autres, les cires de Carnauba, de Candelilla, d'Ouricurry, les cires de fibres de liège, les cires de canne à sucre et les cires du Japon. Parmi les cires minérales, on peut citer, en particulier, les paraffines, les cires mirocristallines, les cires de lignite (Montant wachs) et les ozokérites. Parmi les cires synthétiques, on peut citer, en particulier, les cires de polyéthylène, les cires obtenues par synthèse de Fischer et Tropsch, et les polymères cireux ainsi que leurs esters. Toutes ces cires sont bien connues de l'homme de l'art.

Parmi les cires ayant un point de fusion inférieur à 55°C, on peut citer les cires minérales telles que la vaseline ; les huiles hydrogénées concrètes à 25°C telles que l'huile de ricin hydrogénée, l'huile de palme hydrogénée, le suif hydrogéné, l'huile de coco hydrogénée ; les esters gras concrets à 25°C tels que le myristate de propylèneglycol et le myristate de myristyle, les mono-, di-, et triglycérides concrets à 25°C, la colophane et ses dérivés, l'alcool cétylique, les oléates, myristates, lanolates, stéarates et dihydroxystéarates de calcium, de magnésium, de zinc et d'aluminium.

Parmi les huiles susceptibles d'être utilisées en mélange avec les cires, on peut en particulier citer :
- les huiles minérales telles que l'huile de paraffine, l'huile de vaseline et les huiles minérales ayant un point d'ébullition compris entre 310 et 410 °C.
- les huiles d'origine animale telles que le perhydrosqualène,
- les huiles végétales telles que l'huile d'amande douce, l'huile de calophyllum, l'huile de palme, l'huile d'avocat, l'huile de jojoba, l'huile d'olive, l'huile de ricin, les huiles de germes de céréales telles que l'huile de germes de blé,
- les huiles de silicone telles que le diméthylpolysiloxane,
- les huiles perfluorées telles que les "FOMBLINS" de la Société MONTEFLUOS,
- les esters de synthèse tels que l'huile de Purcelin, le myristate de butyle, le myristate d'isopropyle, le myristate de cétyle, le palmitate d'isopropyle, le stéarate de butyle, le stérate d'hexadécyle, le stéarate d'isopropyle, le stéarate d'octyle, le stéarate d'isocétyle, l'oléate de décyle, le laurate d'hexyle, le dicaprylate de propylène glycol, l'adipate de di-isopropyle,
- les alcools organiques tels que l'alcool oléique, l'alcool linoléique, l'alcool linolénique, l'alcool isostéarylique, l'octyl dodécanol,
- les esters dérivés d'acide lanolique tels que le lanolate d'isopropyle, le lanolate d'isocétyle,
- parmi les huiles on peut également citer : les acétylglycérides, les octanoates et décanoates d'alcools et de polyalcools tels que ceux de glycol et de glycérol, les ricinoléates d'alcools et de polyalcools tels que celui de cétyle.

Le corps gras peut éventuellement contenir des pigments :
Comme pigments colorés, on peut mentionner le noir de carbone ou l'oxyde de fer noir, les oxydes de chrome, les oxydes de fer jaune et rouge, les outremers (polysulfures d'aluminosilicates), le pyrophosphate de manganèse, le bleu ferrique, le bioxyde de titane et enfin certaines poudres métalliques telles que celles d'argent ou d'aluminium. Les pigments sont le plus souvent utilisés en mélange avec des agents nacrants tels que l'oxychlorure de bismuth, le mica-titane, les cristaux de guanine et certains colorants organiques tels que le carmin de cochenille et les laques organiques.

Ces laques qui sont couramment employées pour conférer aux lèvres et à la peau un effet de maquillage, sont des sels de calcium, de baryum, d'aluminium ou de zirconium, de colorants acides tels que les colorants halogéno-acides, azoiques, anthraquinoniques, etc.

Parmi ces laques, on peut en particulier citer celles connues sous les dénominations de D and C Red 21, D and C Orange 5, D and C Red 27, D and C Orange 10, D and C Red 3, D and C Red 7, D and C Red 2, D and C Red 4, D and C Red 8, D and C Red 33, D and C Yellow 5, D and C Yellow 6, D and C Green 5, D and C Yellow 10, D and C Green 3, D and C Blue 1, D and C Blue 2, D and C Violet 1, etc...

Lorsque les dispersions se présentent sous forme de produits de maquillage celles-ci peuvent également contenir des agents antioxydants à raison de 0 à 3 %, de préférence de 0,05 à 0,5 %, tels que les esters propylique, octylique et dodécylique de l'acide gallique, le butylhydroxytoluène, et le butylhydroxyanisole ainsi que des parfums et des agents conservateurs tels que le parahydroxybenzoate de méthyle ou de propyle. Ces additifs se trouvent selon leur solubilité soit dans la phase grasse soit dans la phase dispersée d'alcool polyhydrique.

Le corps gras peut véhiculer un ou plusieurs actifs liposolubles communément utilisés dans les produits cosmétiques ou pharmaceutiques, à raison de 0,05 à 5 % et de préférence de 0,5 à 3 %.

Parmi ceux-ci, on peut citer les dérivés de vitamines tels que l'acétate de tocophérol et le palmitate de vitamine A, les acides gras essentiels, les sphingocérils et les filtres solaires solubles.

Parmi les tensioactifs habituellement utilisés dans les rouges à lèvres, on peut mentionner les tensioatifs anioniques ou non ioniques, de HLB inférieur à 10 (préférentiellement inférieur à 5) à l'exclusion des tensioactifs siliconés. Parmi les non ioniques, on peut citer les lécithines, les succinylglycérides, les alkylphosphates.

Parmi les tensioactifs anioniques, on peut citer le lanolate de magnésium, le lanolate de zinc, le lanolate de cuivre, le lanolate d'arginine et l'octadécanoate de magnésium.

De manière générale, le procédé selon l'invention comprend les étapes suivantes :
On chauffe la phase grasse constituée de 10 à 50 % d'au moins une cire de point de fusion supérieur à 55°C, et contenant éventuellement en dispersion des pigments et/ou des charges, à une température supérieure à la température la plus élevée de fusion des cires (température finissante), et on chauffe d'autre part l'alcool polyhydrique contenant éventuellement des additifs solubles à la même température et on mélange les deux constituants chauffés à l'aide d'une turbine ayant une vitesse de rotation comprise entre 1500 et 3500 t/mn et de préférence entre 2500 et 3500 t/mn pour produire la dispersion. Celle-ci est ensuite coulée dans un moule approprié.

La turbine utilisable dans le procédé selon l'invention peut être de tout modèle connu dans le commerce pour la préparation des compositions cosmétiques ou pharmaceutiques. On citera par exemple la turbine MORITZ.

Afin de déterminer la taille des particules dispersées selon l'invention, on peut utiliser toute technique prévue appropriée, telle que la cryofracture ou la microscopie optique.

Les exemples suivants illustrent l'invention d'une manière non limitative :

### EXEMPLE 1

On a préparé selon l'invention, une dispersion solide sous forme de bâton de rouge à lèvres ayant la composition suivante :

| | |
|---|---|
| - Huile de ricin | 3 % |
| - Huile de vaseline | 9 % |
| - Lanoline | 15 % |
| - Butylhydroxytoluène (BHT) | 0,2 % |
| - Cire d'abeilles | 8,8 % |
| - Ozokérite | 10 % |
| - Lécithine de soja | 4 % |
| - Glycérine | 10 % |
| - Polyéthylène glycol 300 | 12 % |
| - Talc | 3 % |
| - D & C Red 27 | 5 % |
| - Oxyde de fer noir | 2 % |
| - D & C Red 7 | 6 % |
| - Polybutène (INDOPOL de la Société Amoco) | 12 % |
| - Parfum | qs |

Le bâton est préparé de la façon suivante :

On chauffe la phase grasse constituée de l'huile de ricin, l'huile de vaseline, la lanoline, le butylhydroxytoluène (BHT), la cire d'abeilles, l'ozokérite, la lécithine de soja et le polybutène ; les colorants sont broyés dans la phase grasse, puis on ajoute le talc. Par ailleurs, on chauffe le mélange constitué de la glycérine et du polyéthylèneglycol 300, auquel on ajoute le parfum ; on utilise une turbine MORITZ à la vitesse de rotation de 3000 t/mn pour mélanger la phase grasse et la phase alcoolique jusqu'à l'obtention d'une composition de consistance apte à être coulée, tout en refroidissant.

La taille moyenne des particules dispersées est comprise entre 0,03 et 0,5 »m.

### EXEMPLE 2

On a préparé selon l'invention, une dispersion solide sous forme de bâton ayant la composition suivante :

| | |
|---|---|
| - Huile de ricin | 20 % |
| - Huile de jojoba | 20 % |
| - Lanolate d'isopropyle | 27,9 % |
| - BHT | 0,1 % |
| - Cire microcristalline | 8 % |
| - Cire de carnauba | 8 % |
| - Phosphate de tri-oléyle (HOSTAPHAT KO 3000 de Hoechst) | 1 % |
| - Glycérine | 15 % |
| - Pigments | qs |

Le bâton est préparé comme pour l'exemple 1.

La taille moyenne des particules est comprise entre 0,03 et 0,5 »m.

### EXEMPLE 3

On a préparé selon l'invention, une dispersion solide sous forme de bâton ayant la composition suivante :

| | |
|---|---|
| - Huile de ricin | 17 % |
| - Huile de jojoba | 17 % |
| - Lanolate d'isopropyle | 23,9 % |
| - BHT | 0,1 % |
| - Cire microcristalline | 4 % |
| - Cire de carnauba | 4 % |
| - Phosphate de tri-oléyle (HOSTAPHAT KO 300 de Hoechst) | 1 % |
| - Glycérine | 15 % |
| - Polylaurate de vinyle | 10 % |
| - Polyéthylène | 8 % |
| - Pigments | qs |

Le bâton est préparé comme pour l'exemple 1.

La taille moyenne des particules est comprise entre 0,03 et 0,5 »m.

### EXEMPLE 4

On a préparé selon l'invention une dispersion solide coulée pour fard à joues ayant la composition suivante :

| | |
|---|---|
| - Huile de vaseline | 30,65 % |
| - Petrolatum | 10 % |
| - Cire de carnauba | 5 % |
| - Copolymère d'éthylène et de dérivés acryliques | 5 % |
| - Mica-titane | 5 % |
| - Oxyde de titane | 5 % |
| - Talc | 5 % |
| - D & C Red 7 | 0,2 % |
| - Oxyde de fer brun | 2 % |
| - Polybutène | 10 % |
| - BHT | 0,05 % |
| - Para-hydroxybenzoate de propyle | 0,1 % |
| - Glycérine | 20 % |
| - Lécithine | 2 % |

Le bâton est préparé comme pour l'exemple 1.

La taille moyenne des particules est comprise entre 0,03 et 0,5 »m.

### EXEMPLE 5

On a préparé selon l'invention une dispersion solide coulée pour fond de teint ayant la composition suivante :

| | |
|---|---|
| - Cire microcristalline | 8 % |
| - Cire de carnauba | 4 % |
| - Octyl palmitate | 14,5 % |
| - Isoparaffine | 23 % |
| - Lanolate d'isopropyle | 4 % |
| - Para-hydroxybenzoate de propyle | 0,2 % |
| - Oxyde de fer jaune | 1 % |
| - Oxyde de fer brun | 0,37 % |
| - Oxyde de fer noir | 0,15 % |
| - Oxyde de titane | 5,47 % |
| - Oxyde de zinc | 3 % |
| - Talc | 3 % |
| - Poudre de nylon | 3 % |
| - Microsphères vendues sous la dénomination commerciale de "Expancel DE" par la Société KEMANORD PLAST AB | 1,2 % |
| - Diméthicone | 0,3 % |
| - Polylaurate de vinyle | 12,5 % |
| - Glycérine | 15 % |
| - Lanolate de magnésium | 1 % |
| - Parfum | 0,31 % |

Le fond de teint coulé est préparé comme pour l'exemple 1.

La taille moyenne des particules est comprise entre 0,03 et 0,5 »m.

### EXEMPLE 6

On a préparé selon l'invention une dispersion solide coulée pour fond de teint ayant la composition suivante :

| | |
|---|---|
| - Triglycérides des acides caprylique et caprique (Miglyol 810 de la Société HULS) | 3 % |
| - Huile de Sésame | 10 % |
| - Lanoline acétylée | 8 % |
| - Cire de Candellila | 8 % |
| - Cire microcristalline | 12 % |
| - Lanolate de Magnésium | 5 % |
| - Polyglycérine 500 | 20 % |
| - FD & C Yellow 6 | 5 % |
| - D & C Red 7 | 5 % |
| - Dioxide de titane | 2 % |
| - Polylaurate de vinyle | 15 % |
| - Polystéaryl méthylsiloxane (Abil wax 9000 de la Société GOLDSCHMIDT) | 5 % |
| - Butylhydroxytoluène (BHT) | 0,2 % |
| - Parfum | qs |

Le fond de teint est préparé comme pour l'exemple 1.

La taille moyenne des particules est comprise entre 0,03 et 0,5 »m.

## Revendications

1. Procédé de préparation d'une dispersion solide anhydre et stable contenant de 20 à 95 % d'un corps gras constitué de 10 à 50 % d'au moins une cire de point de fusion supérieur à 55°C et de 4 à 50 % d'un alcool polyhydrique, caractérisé par le fait que l'on chauffe le corps gras et l'alcool polyhydrique à une température comprise entre 65 et 95°C et que l'on procède au mélange à l'aide d'une turbine ayant une vitesse de rotation supérieure à 1500 t/mn, de préférence comprise entre 2500 et 3500 t/mn.

2. Procédé selon la revendication 1 caractérisé par le fait que le corps gras représente de 40 à 85 % et l'alcool polyhydrique de 6 à 40 %.

3. Procédé selon la revendication 1, cractérisé par le fait que le corps gras est constitué de 10 à 50 % d'au moins une cire de point de fusion supérieur à 55°C, le reste étant soit une cire de point de fusion inférieur à 55°C soit une huile ou un mélange de ceux-ci.

4. Procédé selon les revendications 1 à 3, caractérisé par le fait que l'on chauffe d'une part la phase grasse, éventuellement contenant en dispersion des pigments, à une température supérieure à la température la plus élevée de fusion des cires, que l'on chauffe d'autre part l'alcool polyhydrique à la même température et qu'on les mélange à l'aide d'une turbine ayant une vitesse de rotation comprise entre 1500 et 3500 t/mn.

5. Dispersion solide anhydre et stable comprenant de 20 à 95 % et de préférence de 40 à 85 %, d'un corps gras et de 4 à 50 % et de préférence de 6 à 40 % d'un alcool polyhydrique, caractérisée par le fait que la taille moyenne des particules d'alcool polyhydrique dispersé est inférieure ou égale à 1 »m. et que le corps gras est constitué de 10 à 50 % en poids d'au moins une cire de point de fusion supérieur à 55°C.

6. Dispersion selon la revendication 5, caractérisé par le fait que l'alcool polyhydrique est un composé ayant de 2 à 8 atomes de carbone et de 2 à 6 fonctions hydroxy.

7. Dispersion selon la revendication 6, caractérisée par le fait que l'alcool polyhydrique est choisi parmi l'éthylène glycol, la glycérine, le propanediol-1,2, la diglycérine, l'érythritol, l'arabitol, l'adonitol, le sorbitol, le dulcitol et leurs mélanges.

8. Dispersion selon la revendication 5, caractérisée par le fait que l'alcool polyhydrique est un polyéther alcool de poids moléculaire moyen compris entre 150 et 600, choisi parmi le polyéthylène glycol 300 et la polyglycérine 500 et leurs mélanges.

9. Dispersion selon l'une quelconque des revendications 5 à 8, caractérisée par le fait qu'elle contient de 0,01 à 20 % et de préférence de 0,5 à 10 % d'un tensioactif hydrocarbone.

10. Dispersion selon l'une quelconque des revendications 5 à 9, caractérisée par le fait que l'alcool polyhydrique contient des actifs hydrosolubles à raison de 0,05 à 5 % par rapport au poids total de la dispersion.

11. Dispersion selon l'une quelconque des revendications 5 à 10, caractérisée par le fait qu'elle contient au moins un polymère susceptible de la stabiliser en atmosphère humide.

12. Dispersion selon la revendication 11, caractérisée par le fait qu'elle contient une quantité d'alcool polyhydrique tel que le rapport en poids du polymère à l'alcool polyhydrique soit inférieur à 4, et est de préférence compris entre 0,25 et 2.

13. Dispersion selon les revendications 11 et 12, caractérisée par le fait que le polymère est choisi parmi les polyalkylènes, les polyacrylates et les polymères siliconés.

14. Dispersion selon l'une quelconque des revendications 5 à 13, caractérisée par le fait qu'elle contient jusqu'à 30 %, et de préférence de 0,1 à 20 % d'un pigment.

15. Dispersion selon l'une quelconque des revendications 5 à 14, caractérisée par le fait qu'elle contient jusqu'à 10 % d'une charge minérale.

## Claims

1. Process for the preparation of a stable, anhydrous, solid dispersion containing from 20 to 95% of a fatty substance consisting of 10 to 50% of at least one wax with a melting point greater than 55°C and from 4 to 50% of a polyhydric alcohol, characterised in that the fatty substance and the polyhydric alcohol are heated to a temperature of between 65 and 95°C and in that mixing is carried out using a turbine having a rotational speed greater than 1500 r/min, preferably between 2500 and 3500 r/min.

2. Process according to Claim 1, characterized in that the fatty substance represents from 40 to 85% and the polyhydric alcohol from 6 to 40%.

3. Process according to Claim 1, characterized in that the fatty substance consists of 10 to 50% of at least one wax with a melting point greater than 55°C, the remainder being either a wax with a melting point of less than 55°C or an oil or a mixture of the latter.

4. Process according to Claims 1 to 3, characterized in that, on the one hand, the fatty phase, optionally containing dispersed pigments, is heated to a temperature greater than the highest melting temperature of the waxes, in that, on the other hand, the polyhydric alcohol is heated to the same temperature and in that they are mixed using a turbine having a rotational speed of between 1500 and 3500 r/min.

5. Stable, anhydrous, solid dispersion comprising from 20 to 95% and preferably from 40 to 85% of a fatty substance and from 4 to 50% and preferably from 6 to 40% of a polyhydric alcohol, characterized in that the mean size of the particles of dispersed polyhydric alcohol is less than or equal to 1 »m and in that the fatty substance consists of 10 to 50% by weight of at least one wax with a melting point greater than 55°C.

6. Dispersion according to Claim 5, characterized in that the polyhydric alcohol is a compound having from 2 to 8 carbon atoms and from 2 to 6 hydroxyl functional groups.

7. Dispersion according to Claim 6, characterized in that the polyhydric alcohol is chosen from ethylene glycol, glycerol, 1,2-propanediol, diglycerol, erythritol, arabitol, adonitol, sorbitol, dulcitol and their mixtures.

8. Dispersion according to Claim 5, characterized in that the polyhydric alcohol is a polyether alcohol with a mean molecular weight of between 150 and 600 chosen from polyethylene glycol 300 and polyglycerol 500 and their mixtures.

9. Dispersion according to any one of Claims 5 to 8, characterized in that it contains from 0.01 to 20% and preferably from 0.5 to 10% of a hydrocarbon surface-active agent.

10. Dispersion according to any one of claims 5 to 9, characterized in that the polyhydric alcohol contains water-soluble active ingredients in a proportion of 0.05 to 5% with respect to the total weight of the dispersion.

11. Dispersion according to any one of Claims 5 to 10, characterized in that it contains at least one polymer capable of stabilizing it in a moist atmosphere.

12. Dispersion according to Claim 11, characterized in that it contains an amount of polyhydric alcohol such that the ratio by weight of the polymer to the polyhydric alcohol is less than 4 and is preferably between 0.25 and 2.

13. Dispersion according to Claims 11 and 12, characterized in that the polymer is chosen from polyalklenes, polyacrylates and silicone polymers.

14. Dispersion according to any one of Claims 5 to 13, characterized in that it contains up to 30%, and preferably from 0.1 to 20%, of a pigment.

15. Dispersion according to any one of Claims 5 to 14, characterized in that it contains up to 10% of an inorganic filler.

## Patentansprüche

1. Verfahren zur Herstellung einer wasserfreien und stabilen Feststoffdispersion mit einem Gehalt an 20 bis 95 % Fettkörper, welcher zu 10 bis 50 % aus mindestens einem Wachs mit einem Schmelzpunkt oberhalb von 55 °C besteht, und 4 bis 50 % Polyalkohol, wobei das Verfahren dadurch gekennzeichnet ist, daß man den Fettkörper und den Polyalkohol auf eine zwischen 65 und 95 °C gelegene Temperatur erwärmt und mit Hilfe einer Turbine mit einer Rotationsgeschwindigkeit von mehr als 1500 UpM, vorzugsweise zwischen 2500 und 3500 UpM, zu einer Mischung verarbeitet.

2. Verfahren gemäß Anspruch 1, dadurch gekennzeichnet, daß der Fettkörper zwischen 40 und 85 % und der Polyalkohol zwischen 6 und 40 % der Dispersion ausmacht.

3. Verfahren gemäß Anspruch 1, dadurch gekennzeichnet, daß der Fettkörper zu 10 bis 50 % aus mindestens einem Wachs mit einem Schmelzpunkt oberhalb von 55 °C besteht, wobei der verbleibende Rest entweder ein Wachs mit einem Schmelzpunkt unter 55 °C oder ein Öl oder ein Gemisch dieser beiden darstellt.

4. Verfahren gemäß einem der Ansprüche 1 bis 3, dadurch gekennzeichnet, daß man zum einen die Fettphase, welche gegebenenfalls darin dispergierte Pigmente enthält, auf eine Temperatur erwärmt, die oberhalb der höchsten Schmelztemperatur der Wachse liegt, und zum anderen den Polyalkohol auf dieselbe Temperatur erwärmt und diese mit Hilfe einer Turbine mit einer zwischen 1500 und 3500 UpM liegenden Rotationsgeschwindigkeit vermischt.

5. Wasserfreie und stabile Feststoffdispersion mit einem Gehalt an 20 bis 95 % und vorzugsweise 40 bis 85 % Fettkörper und 4 bis 50 %, vorzugsweise 6 bis 40 % Polyalkohol, welche dadurch gekennzeichnet ist, daß die mittlere Größe der dispergierten Polyalkoholteilchen maximal 1 »m beträgt und der Fettkörper zu 10 bis 50 Gew.-% aus mindestens einem Wachs mit einem Schmelzpunkt oberhalb von 55 °C besteht.

6. Dispersion gemäß Anspruch 5, dadurch gekennzeichnet, daß der Polyalkohol eine Verbindung mit 2 bis 8 Kohlenstoffatomen und 2 bis 6 Hydroxylfunktionen darstellt.

7. Dispersion gemäß Anspruch 6, dadurch gekennzeichnet, daß der Polyalkohol aus Ethylenglykol, Glycerin, 1,2-Propapdiol, Diglycerin, Erythrit, Arabit, Adonit, Sorbit, Dulcit und deren Gemischen ausgewählt ist.

8. Dispersion gemäß Anspruch 5, dadurch gekennzeichnet, daß der Polyalkohol einen Polyetheralkohol mit einem mittleren Molekulargewicht von 150 bis 600 darstellt und aus Polyethylenglykol 300 und Polyglycerin 500 sowie deren Gemischen ausgewählt ist.

9. Dispersion gemäß einem der Ansprüche 5 bis 8, dadurch gekennzeichnet, daß sie zwischen 0,01 und 20 %, vorzugsweise 0,5 bis 10 %, eines oberflächenaktiven Kohlenwasserstoffes enthalt.

10. Dispersion gemäß einem der Ansprüche 5 bis 9, dadurch gekennzeichnet, daß der Polyalkohol wasserlösliche Wirkstoffe in einer Menge von 0,05 bis 5 % enthält, bezogen auf das Gesamtgewicht der Dispersion.

11. Dispersion gemäß einem der Ansprüche 5 bis 10, dadurch gekennzeichnet, daß sie mindestens ein zu ihrer Stabilisierung in feuchter Atmosphäre geeignetes Polymer enthält.

12. Dispersion gemäß Anspruch 11, dadurch gekennzeichnet, daß sie eine solche Menge an Polyalokohol enthält, daß das Gewichtsverhältnis von Polymer zu Polyalkohol Kleiner als 4 ist und vorzugsweise zwischen 0,25 und 2 liegt.

13. Dispersion gemäß Anspruch 11 und 12, dadurch gekennzeichnet, daß das Polymer unter Polyalkylenen, Polyacrylaten und Silikonpolymeren ausgewählt ist.

14. Dispersion gemäß einem der Ansprüche 5 bis 13, dadurch gekennzeichnet, daß sie bis zu 30 % und vorzugsweise zwischen 0,1 und 20 % eines Pigments enthält.

15. Dispersion gemäß einem der Ansprüche 5 bis 14, dadurch gekennzeichnet, daß sie bis zu 10 % eines mineralischen Zusatzes enthält.
